# EUROPÄISCHE PATENTANMELDUNG

(11) **EP 3 782 622 A1**
(43) Veröffentlichungstag der Anmeldung: **24.02.2021**
(21) Anmeldenummer: 20191883.6
(22) Anmeldetag: 20.08.2020
(51) Int. Cl.: A61K 31/44, A61P 21/00, A61P 21/06

(54) **ADENOSIN A2B-REZEPTOR-AGONIST ZUR BEHANDLUNG VON KRANKHEITEN DES MUSKEL-SKELETT-SYSTEMS**

(30) Priorität: 21.08.2019 DE 102019122497
(71) Anmelder: Rheinische Friedrich-Wilhelms-Universität Bonn, 53113 Bonn (DE)
(72) Erfinder: Pfeifer, Prof. Dr., Alexander, 53125 Bonn (DE); Fleischmann, Prof. Dr., Bernd, 53125 Bonn (DE); Wenzel, Dr., Daniela, 53121 Bonn (DE); Naumann, Dr., Jennifer, 50354 Hürth (DE); Haufs-Brusberg, Dr., Saskia, 53111 Bonn (DE); Hochhäuser, Dr., Aileen, 53757 Sankt Augustin (DE); Reverte-Salisa, Dr., Laia, 53111 Bonn (DE); Copperi, Francesca, 53127 Bonn (DE); Gnad, Dr., Thorsten, 53332 Bornheim (DE)
(74) Vertreter: Kutzenberger Wolff & Partner

(57) **Zusammenfassung**

Die vorliegende Erfindung betrifft neue Behandlungsstrategien von Krankheiten des Muskel-Skelett-Systems und des Bindegewebes, insbesondere von Sarkopenie, welche auf der Aktivierung des Adenosin-A_{2B}-Rezeptors basieren.

## Beschreibung

Die vorliegende Erfindung betrifft neue Behandlungsstrategien von Krankheiten und Verletzungen des Muskel-Skelett-Systems und des Bindegewebes, insbesondere von Sarkopenie, welche auf der Aktivierung des Adenosin-A_{2B}-Rezeptors basieren.

Sarkopenie ist ein geriatrisches Syndrom und bezeichnet den mit fortschreitendem Alter zunehmenden Abbau von Masse und Kraft des Skelettmuskels (SKM) und die damit einhergehenden funktionellen Einschränkungen des älteren Menschen (A. A. Sayer, H. Syddall, H. Martin, H. Patel, D. Baylis, C. Cooper, J. Nutr. Health Aging 2008, 12, 427-432). Sarkopenie erhöht das Risiko von Stürzen, reduziert die Lebensqualität und geht mit einer erhöhten Sterblichkeit einher. Obwohl es sich bei Sarkopenie in erster Linie um eine Krankheit der älteren Menschen handelt, kann ihre Entwicklung mit Erkrankungen verbunden sein, die nicht ausschließlich bei älteren Menschen auftreten. Sarkopenie erhöht das Risiko einer körperlichen Einschränkung und nachfolgender Invalidität; neuere Untersuchungen zeigen auch, dass diese Erkrankung das Risiko von komorbiden Erkrankungen wie beispielsweise Osteoporose, Adipositas, oder Typ 2 Diabetes erhöht.

Sarkopenie ist jedoch nicht nur mit der Entwicklung von körperlicher Gebrechlichkeit und Behinderung verbunden (I. Janssen, S. B. Heymsfield, R. Ross, J. Am. Geriatr. Soc. 2002, 50, 889-896), sondern hat auch wichtige metabolische Folgen: Die Skelettmuskulatur ist nicht nur für den Energieaufwand (EE) bei körperlicher Aktivität verantwortlich, sondern trägt auch wesentlich zum Energieaufwand im Ruhezustand bei. Folglich kann eine verminderte Muskelfunktion zu einem Ungleichgewicht der energetischen Homöostase und Adipositas führen (J. A. Batsis, D. T. Villareal, Nat. Rev. Endocrinol. 2018, 14, 513-537). Aus diesem Grund ist das Altern mit einer Zunahme der abdominalen Adipositas verbunden (M. Jura, L. P. Kozak, Age 2016, 38, 23.) und die Prävalenz von Adipositas in Kombination mit Sarkopenie (d.h. sarkopenischer Adipositas) steigt bei Erwachsenen ab 65 Jahren (J. A. Batsis, D. T. Villareal, Nat. Rev. Endocrinol. 2018, 14, 513-537).

Sarkopenie und sarkopenische Adipositas sind hochriskante geriatrische Syndrome, die mit einem erhöhten Risiko für unfall- oder krankheitsbedingter Invalidität und Immobilität verbunden sind (J. A. Batsis, D. T. Villareal, Nat. Rev. Endocrinol. 2018, 14, 513-537) und stellen als solche per se bereits eine enorme Belastung für die menschliche Gesundheit dar. Darüber hinaus ist es mit einem erhöhten Sterberisiko, Krankenhausaufenthalten, Pflegebedarf und höheren Gesundheitsausgaben verbunden (V. Santilli, A. Bernetti, M. Mangone, M. Paoloni, Clinical Cases in Mineral and Bone Metabolism 2014, 11(3), 177-180.).

In Kombination mit einer zunehmend alternden Bevölkerung kann von einer zunehmenden Anzahl an Krankheitsfällen ausgegangen werden die in Folge zu einem weiteren Anstieg der finanziellen Belastung des Gesundheitssystems führen.

Bisherige Ansätze zur Therapie und Prävention von Sarkopenie zielen darauf ab, die Muskelmasse, Muskelkraft und Muskelleistung zu verbessern. Beispiele hierfür sind körperliche Aktivität und das Vermeiden des Rauchens (Prävention) sowie Krafttraining und ernährungsmedizinische Ansätze (Therapie). Ansätze zur kausalen, pharmakologischen Therapie der Sarkopenie sind bisher nur in geringem Umfang erforscht und nicht am Markt verfügbar.

Eine Screening-Methode für Sarkopenie ist beispielsweise SARC-F (Cao L, Chen S, Zou C, Ding X, Gao L, Liao Z, et al. A pilot study of the SARC-F scale on screening sarcopenia and physical disability in the Chinese older people. J Nutr Health Aging 2014; 18:277-283).

WO 2006/099958 A1 bezieht sich auf substituierte 2-Thio-3,5-dicyano-4-phenyl-6-aminopyridine und deren Verwendung in Arzneimitteln zur Prophylaxe und/oder Behandlung von Reperfusionsschäden.

US 2007/0190056 A1 betrifft ein Verfahren zur Behandlung und/oder Verbesserung eines oder mehrerer Symptome von Sarkopenie und altersbedingter Muskeldegeneration bei einem Säuger.

US 2018/282733 A1 betrifft ein therapeutisches Mittel für ein Muskelschwächesymptom (Sarkopenie) oder eine Stoffwechselerkrankung, das eine µ-Crystallin (CRYM)-hemmende Substanz als Wirkstoff enthält. Die hemmende Substanz ist ausgewählt aus der Gruppe bestehend aus einer Antisense-Nukleinsäure gegen CRYM, einer RNAi-induzierenden Nukleinsäure gegen CRYM und einem Ribozym gegen CRYM, Expressionsvektoren davon, einem Antagonisten-Antikörper gegen CRYM und einer niedermolekularen Verbindung, die die Aktivität von CRYM hemmt.

Es besteht ein Bedarf an neuen und vorteilhaften oder verbesserten Behandlungsmöglichkeiten für Krankheiten des Muskel-Skelett-Systems und des Bindegewebes, insbesondere von Sarkopenie.

Diese Aufgabe wird durch den Gegenstand der Patentansprüche gelöst.

Ein erster Aspekt der Erfindung betrifft daher Adenosin-A_{2B}-Rezeptor-Agonisten zur Anwendung bei der Behandlung von Krankheiten des Muskel-Skelett-Systems und des Bindegewebes.

Es wurde überraschend gefunden, dass Adenosin-A_{2B}-Rezeptor-Agonisten zur Behandlung von Krankheiten des Muskel-Skelett-Systems und Bindegewebes, insbesondere Sarkopenie, geeignet sind.

Krankheiten des Muskel-Skelett-Systems und Bindegewebes, insbesondere Sarkopenie, gehen mit einem Verlust von Muskelfunktion einher, u.a. einem Verlust der Funktion von Skelettmuskelzellen. Es wurde überraschend gefunden, dass die pharmakologische Stimulation der Adenosin-A_{2B}-Rezeptoren mit dem Adenosin-A_{2B}-Rezeptor-Agonisten 2-({6-Amino-3,5-dicyano-4-[4-(cyclopropyl-methoxy)phenyl]pyridin-2-yl}sulfa-nyl)-acetamid (BAY 60-6583) diesem Verlust von Muskelfunktion entgegenwirkt.

Die Wirkung des Adenosin-A_{2B}-Rezeptor-Agonisten kann z.B. anhand von Sauerstoffverbrauch und Glukoseaufnahme der Skelettmuskelzellen gemessen werden. Sauerstoffverbrauch und Glukoseaufnahme von Skelettmuskelzellen können also als Maß für die Aktivität und Leistungsfähigkeit von Skelettmuskelzellen betrachtet werden.

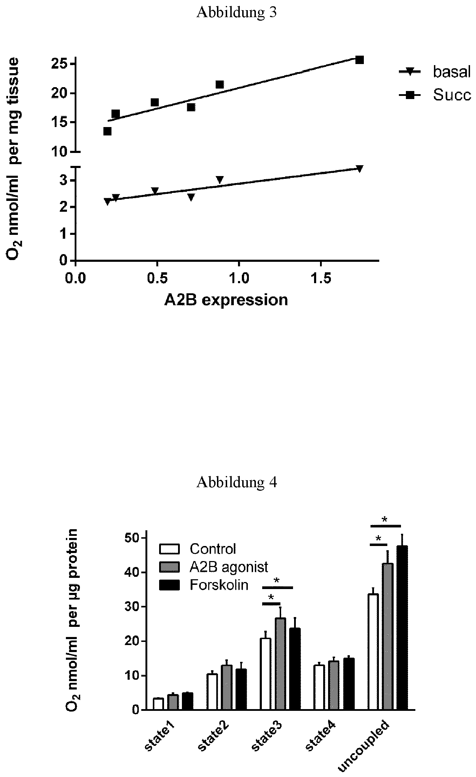

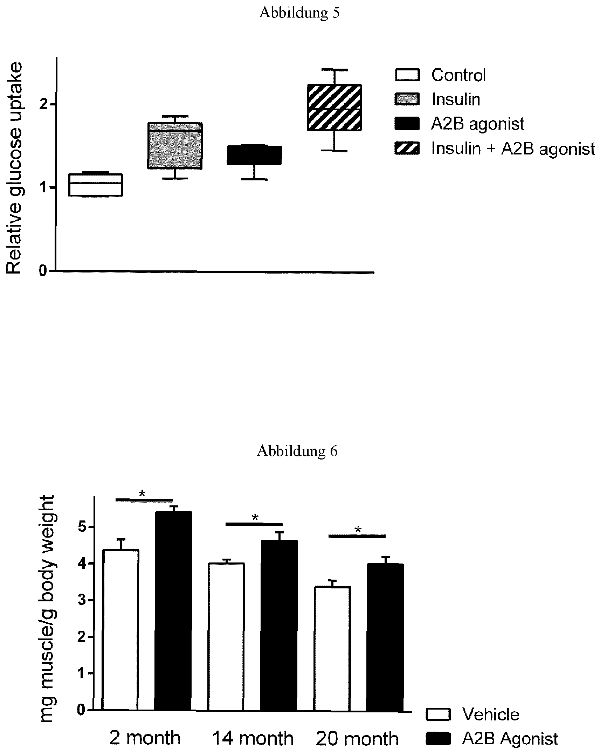

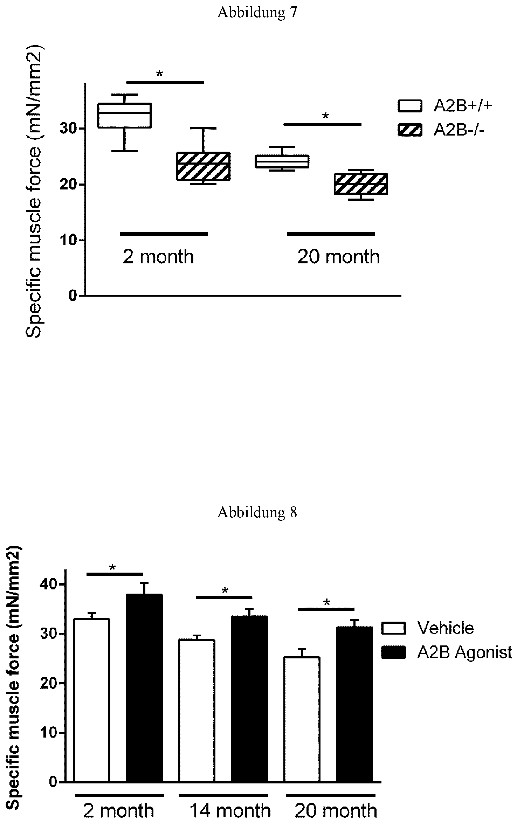

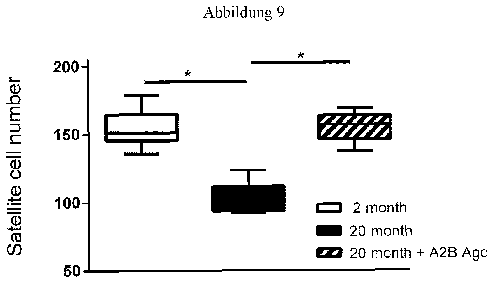

In *in vitro* Experimenten mit humanen Zellen konnte überraschend gezeigt werden, dass die pharmakologische Stimulation der Adenosin-A_{2B}-Rezeptoren mit dem Adenosin-A_{2B}-Rezeptor-Agonisten 2-({6-Amino-3,5-dicyano-4-[4-(cyclo-propylmethoxy)phenyl]pyridin-2-yl}sulfanyl)-acetamid (BAY 60-6583)
(i) den Sauerstoffverbrauch von humanen und murinen Skelettmuskel Explantaten signifikant erhöht (siehe Abb. 1 und Abb. 2); weiterhin konnte gezeigt werden, dass die Expression von A_{2B}-Rezeptoren in humanem Skelettmuskel mit dem basalen Sauerstoffverbrauch korreliert (siehe Abb. 3);
(ii) den Sauerstoffverbrauch von primären humanen Skelettmuskelzellen signifikant erhöht (siehe Abb. 4); und (iii) die Glukoseaufnahme von primären humanen Skelettmuskeln signifikant erhöht (Abb. 5).
   In *in vivo* Experimenten mit Mäusen konnte überraschend gezeigt werden, dass die pharmakologische Stimulation der Adenosin-A_{2B}-Rezeptoren mit dem Adenosin-A_{2B}-Rezeptor-Agonisten 2-({6-Amino-3,5-dicyano-4-[4-(cyclo-propylmethoxy)phenyl]pyridin-2-yl}sulfanyl)-acetamid (BAY 60-6583)
(iv) dem alterungsbedingten Verlust von Muskelmasse entgegenwirkt (Abb. 6);
(v) weiterhin wurde gefunden, dass die Muskelkraft von Mäusen mit einem Defizit an Adenosin-A_{2B}-Rezeptoren im Vergleich zu gleichaltrigen Wildtyp Mäusen signifikant verringert ist (Abb. 7);
(vi) die Muskelkraft von Mäusen signifikant erhöht (Abb. 8); obwohl die Muskelkraft im Alter nachlässt, haben behandelte gealterte Mäuse hierbei so viel Kraft wie junge Kontrolltiere; und
(vii) die Anzahl der muskelspezifischen Stammzellen (Satellitenzellen) erhöht und damit der abnehmenden Regenerationsfähigkeit der Skelettmuskulatur im Alter entgegenwirkt (Abb. 9). Eine Behandlung mit A_{2B} Agonisten sollte daher auch positive Effekte bei der Behandlung von Muskelverletzungen haben, da hierbei die Satellitenzellen eine enorme Bedeutung haben.

Die Erfindung betrifft Adenosin-A_{2B}-Rezeptor-Agonisten. Die *International Union of Pharmacology (IUPHAR)* unterteilt Rezeptoren nach einem Rezeptor-Code (https://www.guidetopharma-cology.org/targets.jsp). Gemäß dieser Einteilung gibt es Subtypen von Adenosinrezeptoren, und zwar A₁, A_{2A}, A₃, und A_{2B}. Die Erfindung betrifft Agonisten des A_{2B}-Subtyps.

Informationen u. a. zum Adenosin-A_{2B}-Rezeptor finden sich beispielsweise in (i) Fredholm, B. B.; AP, I. J.; Jacobson, K. A.; Klotz, K. N.; Linden, J. International Union of Pharmacology. XXV. Nomenclature and classification of adenosine receptors. Pharmacol. Rev. 2001, 53, 527-552 und (ii) Linden, J.; Thai, T.; Figler, H.; Jin, X.; Robeva, A. S. Characterization of human A2B adenosine receptors: radioligand binding, western blotting, and coupling to Gq in human embryonic kidney 293 cells and HMC-1 mast cells. Mol. Pharmacol. 1999, 56, 705-713.

Adenosin-A_{2B}-Rezeptor-Agonisten sind also bekannt und u.a. auch kommerziell verfügbar. Erfindungsgemäß ist als Adenosin-A_{2B}-Rezeptor-Agonist grundsätzlich jede Substanz aufzufassen, welche den Adenosin-A_{2B}-Rezeptor agonisiert und bevorzugt einen IC₅₀ Wert von höchstens 100 µM, bevorzugter höchstens 10 µM, noch bevorzugter höchstens 1000 nM, am bevorzugtesten höchstens 500 nM, insbesondere höchstens 250 mM aufweist. Experimentelle Details zur Bestimmung des IC₅₀ Werts sind einem Fachmann bekannt. Diesbezüglich wird beispielsweise vollumfänglich verwiesen auf J. Goulding et al., Biochem Pharmacol. 2018 Jan; 147: 55-66. doi: 10.1016/j.bcp.2017.10.013.

Die zuvor genannte Rezeptor-Selektivität lässt sich bestimmen durch die Wirkung der Substanzen an Zelllinien, welche nach stabiler Transfektion mit der entsprechenden cDNA die jeweiligen Rezeptorsubtypen exprimieren (M.E. Olah, H. Ren, J. Ostrowski, K.A. Jacobson, G.L. Stiles, "Cloning, expression, and characterization of the unique bovine A1 adenosine receptor. Studies on the ligand binding site by site-directed mutagenesis.", J. Biol. Chem. 267 (1992), 10764-10770).

Die Wirkung der Substanzen an solchen Zelllinien lässt sich wiederum erfassen durch biochemische Messung des intrazellulären Botenstoffes cAMP (K.N. Klotz, J. Hessling, J. Hegler, C. Owman, B. Kull, B.B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), 1-9). A_{2B}-Agonisten (Kopplung bevorzugt über Gs-Proteine) führen zu einer Zunahme und A_{2B}-Antagonisten zu einer Abnahme im cAMP-Gehalt der Zellen.

Erfindungsgemäß bevorzugte Adenosin-A_{2B}-Rezeptor-Agonisten sind
(a) BAY 60-6583 (= 2-({6-Amino-3,5-dicyano-4-[4-(cyclopropylmethoxy)-phenyl]pyridin-2-yl}sulfanyl)-acetamid, CAS-Nr. 910487-58-0): 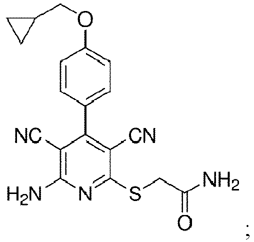
(b) NECA (= 1-(6-Amino-9H-purin-9-yl)-1-deoxy-N-ethyl-β-D-ribo-furanuronamid, CAS-Nr. 35920-39-9,): 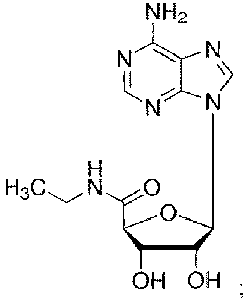
(c) Adenosin (= 9-β-D-Ribofuranosyladenin, CAS-Nr. 58-61-7): 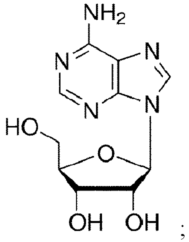
(d) LUF-5834 (= 2-Amino-4-(4-hydroxyphenyl)-6-(1H-imidazol-2-ylmethylsulfanyl)pyridin-3,5-dicarbonitril, CAS-Nr. 333962-91-7): 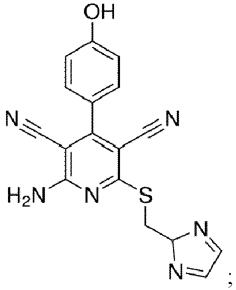 und
(e) CADO (= 6-Amino-2-chloropurin ribosid, CAS-Nr. 146-77-0): 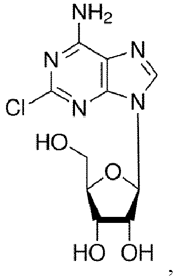 sowie deren physiologisch verträgliche Salzen und/oder Solvate.

Beispiele für geeignete physiologisch verträgliche Salze umfassen Salze anorganischer Säuren, wie z.B. Chlorwasserstoff, Bromwasserstoff und Schwefelsäure, und Salze organischer Säuren, wie Methansulfonsäure, Fumarsäure, Maleinsäure, Essigsäure, Oxalsäure, Bernsteinsäure, Äpfelsäure, Weinsäure, Mandelsäure, Milchsäure, Zitronensäure, Glutaminsäure, Acetylsalicylsäure, Nikotinsäure, Aminobenzoesäure, α-Liponsäure, Hippursäure und Asparaginsäure. Ein nicht einschränkendes Beispiel für ein Solvat ist das Hydrat.

Die Erfindung betrifft außerdem die Behandlung von Krankheiten des Muskel-Skelett-Systems und Bindegewebes, insbesondere Sarkopenie.

Erfindungsgemäße Krankheiten des Muskel-Skelett-Systems und des Bindegewebes sind in Kapitel XIII des ICD-10 Klassifikationssystems für medizinische Diagnosen (https://icd.who.int/en/) aufgeführt. Sarkopenie besitzt die ICD-10-Kodierung M62.50.

Erfindungsgemäße bevorzugte Krankheiten des Muskel-Skelett-Systems und des Bindegewebes sind Krankheiten der Weichteilgewebe (ICD-10-Kodierung: M60-M79), noch bevorzugter Krankheiten der Muskeln (ICD-10-Kodierung: M60-M63) und besonders bevorzugt Sarkopenie (ICD-10-Kodierung: M62.50).

Erfindungsgemäß bevorzugt ist die Sarkopenie ausgewählt aus der Gruppe bestehend aus altersabhängiger Sarkopenie, aktivitätsbedingter Sarkopenie, erkrankungsbedingter Sarkopenie, ernährungsbedingter Sarkopenie und Kombinationen davon. Altersabhängiger Sarkopenie ist durch den Alterungsprozess bedingt. Aktivitätsbedingte Sarkopenie ist durch Immobilität und/oder Schwerelosigkeit bedingt. Erkrankungsbedingter Sarkopenie ist durch Organinsuffizienten (z.B. Herz, Lunge, Niere), entzündliche Erkrankungen, maligne Erkrankungen und endokrine Erkrankungen bedingt. Ernährungsbedingter Sarkopenie ist durch ernährungsabhängige Erkrankungen bedingt. Sarkopenie kann hierbei auch durch eine Kombination verschiedener Ursachen bedingt sein.

Erfindungsgemäß bevorzugt ist die Sarkopenie ferner ausgewählt aus der Gruppe bestehend aus Sarkopenie der Schulterregion (insbesondere Klavikula, Skapula, Akromioklavikular-, Schulter- und/oder Sternoklavikulargelenk), des Oberarms (insbesondere Humerus und/oder Ellenbogengelenk), des Unterarms (insbesondere Radius, Ulna und/oder Handgelenk), der Hand (insbesondere Finger, Handwurzel, Mittelhand und/oder Gelenke zwischen diesen Knochen), der Beckenregion und Oberschenkel (insbesondere Becken, Femur, Gesäß, Hüfte, Hüftgelenk und/oder Iliosakralgelenk), der Unterschenkel (insbesondere Fibula, Tibia und/oder Kniegelenk), der Knöchel und des Fußes (insbesondere Fußwurzel, Mittelfuß, Zehen, Sprunggelenk und/oder sonstige Gelenke des Fußes) und Sarkopenie sonstiger Lokalisationen (insbesondere Hals, Kopf, Rippen, Rumpf, Schädel und/oder Wirbelsäule).

In einer besonders bevorzugten Ausführungsform ist die Krankheit Sarkopenie und der Adenosin-A_{2B}-Rezeptor-Agonist 2-({6-Amino-3,5-dicyano-4-[4-(cyclopropylmethoxy)phenyl]pyridin-2-yl}sulfanyl)-acetamid (BAY 60-6583) oder ein physiologisch verträgliches Salz und/oder Solvat davon.

Erfindungsgemäß bevorzugt wird der Adenosin-A_{2B}-Rezeptor-Agonist in einer pharmazeutischen Zusammensetzung als Darreichungsform bereitgestellt, welche über unterschiedliche Routen verabreicht werden kann. Erfindungsgemäß bevorzugt erfolgt die Verabreichung aurikulär, buccal, epidural, dermal, endobronchial, epidermal, inhalativ, intraarteriell, intraartikulär, intraglutäal, intrakardial, intraläsional, intralumbal, intralymphatisch, intramuskulär, intraossär, intraperitoneal, intrakutan, intrathekal, intravasal, intravenös, intravesikal, intravitreal, konjunktival, kutan, nasal/intranasal, oral/peroral, parenteral, periartikulär, perkutan, pulmonal, rektal, subkutan, sublingual oder transdermal.

In einer bevorzugten Ausführungsform wird der Adenosin-A_{2B}-Rezeptor-Agonist systemisch oder lokal verabreicht. Bevorzugt wird der Adenosin-A_{2B}-Rezeptor-Agonist oral, transdermal, intravenös, subkutan, intraperitoneal oder rektal verabreicht.

In einer bevorzugten Ausführungsform wird der Adenosin-A_{2B}-Rezeptor-Agonist einmal täglich, zweimal täglich oder dreimal täglich verabreicht.

Die Herstellung pharmazeutischer Zusammensetzungen und Darreichungsformen erfolgt erfindungsgemäß bevorzugt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine erfindungsgemäße feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die Wirkstoff in homogener Verteilung enthält. Unter einer homogenen Verteilung wird hier verstanden, dass der Wirkstoff gleichmäßig über die gesamte Zusammensetzung verteilt ist, so dass diese ohne weiteres in gleich wirksame Einheitsdosis-Formen, wie Tabletten, Kapseln, Dragees unterteilt werden kann. Die feste Zusammensetzung wird anschließend in Einheitsdosis-Formen unterteilt. Die Tabletten oder Pillen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an Patienten zu verabreichenden Mengen an Adenosin-A_{2B}-Rezeptor-Agonist, vorzugsweise BAY 60-6583, variieren in Abhängigkeit vom Gewicht des Patienten, von der Applikationsart und dem Schweregrad der Erkrankung. In einer bevorzugten Ausführungsform enthält das Medikament den Adenosin-A_{2B}-Rezeptor-Agonisten, vorzugsweise BAY 60-6583, in einer Menge von 10 bis 300 mg, bevorzugter 20 bis 290 mg, noch bevorzugter 30 bis 280 mg, am bevorzugtesten 40 bis 270 mg, als Äquivalentdosis bezogen auf die freie Base.

Die nachfolgenden Beispiele veranschaulichen die Erfindung, sind jedoch nicht einschränkend auszulegen.

WT männliche C57BL/6 Mäuse wurden von Janvier gekauft. A2B-/- Mäuse wurden von M. Idzko, Freiburg, Deutschland, zur Verfügung gestellt. ATA2B-KO (A2Bfloxed/floxed;AdipoCre+) und Con-A2B (A2Bfloxed/floxed;AdipoCre-) Mäuse wurden durch Züchtung von A2Bfloxed/floxed Mäusen (erzeugt von Holger K. Eltzschig) mit Adiponectin-Cre (Jackson Laboratories) Mäusen zur Herstellung von A2Bfloxed;AdipoCre+ heterozygoten Mäusen erzeugt. Heterozygote Mäuse wurden mit A2Bfloxed/floxed gezüchtet. Männliche Wurfgeschwister wurden zufällig experimentellen Gruppen zugeordnet. Halbfettdiät (HFD, 60% Energie aus Fett) und Kontrolldiät wurden von ssniff Spezialdiäten GmbH (Soest, DE) gekauft. Mäuse wurden in einem täglichen Zyklus von 12 Stunden Licht (0600 bis 1800) und 12 Stunden Dunkelheit (1800 bis 0600) bei 23 ± 1°C gehalten und erhielten freien Zugang zu Futter und Wasser. Für die Muskelanalyse/Sarkopenie wurden Mäusen vier Wochen lang einmal täglich intraperitoneal mit 1 mg/kg 2-({6-Amino-3,5-dicyano-4-[4-(cyclopropylmethoxy)-phenyl]-pyridin-2-yl}sulfanyl)-acetamid (Bay 60-6583) injiziert.

Nachfolgend sind die im Rahmen der vorliegenden Offenbarung verwendeten analytischen Methoden erläutert.

### Isolierung der Skelettmuskulatur und Kraftmessung:

*Extensor digitorum longus* (EDL) Muskeln aus den Hinterbeinen von Mäusen wurden von Sehne zu Sehne isoliert, in eine wässrige Pufferlösung aus Krebs-Puffer (118 mM NaCl, 3,4 mM KCl, 0,8 mM MgSO₄, 1,2 mM KH₂PO₄, 11,1 mM Glukose, 25 mM NaHCO₃, 2,5 mM CaCl₂) getaucht und bei Raumtemperatur mit Carbogen (ein Gasgemisch aus 5 Volumenteilen Kohlenstoffdioxid (CO₂) und 95 Volumenteilen Sauerstoff (O₂)) umspült. Die Muskeln wurden zwischen der Klemme eines Hebelarms und dem Haken eines Kraftaufnehmers eines Myographen (SI Instruments) ausgerichtet. Dann wurden die Muskeln gedehnt, bis ein einzelner Impuls während einer Zuckungsstimulation die maximale Kraft hervorrief. Es wurden Zuckungsstimulationen (2 V, 1 ms) für x min aufgezeichnet und die Kraft gemessen. Die gesamte Muskelquerschnittsfläche wurde geschätzt, indem die Muskelmasse durch das Produkt aus der Muskellänge, dem Verhältnis von Muskellänge zu Faserlänge (0,44 bei EDL) und der Dichte des Säugetiermuskels (1,06 mg/mm³) dividiert wurde. Die spezifische Kraft wurde als Kraft/Gesamtquerschnittsfläche (mN/mm²) berechnet.

### In vivo Gewichthebetest:

Nach 12-wöchiger Kontrolldiät (CD) oder Halbfettdiät (HFD) mit täglicher Behandlung mit dem Adenosin-A_{2B}-Rezeptor-Agonisten 2-({6-Amino-3,5-dicyano-4-[4-(cyclopropylmethoxy)-phenyl]-pyridin-2-yl}sulfanyl)-acetamid (Bay 60-6583) wurden Mäuse wie beschrieben einem Gewichthebetest unterzogen (R. M. Deacon, J. Vis. Exp. 2013, 76: 2610.) um die Muskelkraft zu bestimmen. Das Gewicht der Mäuse (Kettenglieder, die mit Stahlwolle verbunden sind) liegt zwischen 15 g und 94 g.

### Messung der endogenen Atmung:

Skelettmuskelzellen oder -gewebe wurden 15 Minuten vor den autographischen Messungen wie angegeben behandelt (Oxygraph 2K; Oroboros Instruments). Die Proben wurden in die Oxygraphenkammer mit 2 ml Inkubationsmedium (0,5 mM Ethylenglycol-bis(aminoethylether)-N,N,N',N'-tetraessigsäure (EGTA), 3 mM MgCl₂ · 6 H₂O, 60 mM K-Lactobionat, 20 mM Taurin, 10 mM KH₂PO₄, 20 mM 2-(4-(2-Hydroxyethyl)-1-piperazinyl)-ethansulfonsäure (HEPES), 110 mM Saccharose und 1 g/l Bovines Serumalbumin (BSA), pH 7,1) gebracht. *In vitro*-Atmungswerte wurden bei Erreichen eines stationären Zustandes aufgezeichnet, gefolgt von der Zugabe von Substraten (Zustand 1: endogen; Zustand 2: Adenosindiphosphat (ADP); Zustand 3: Succinat; Zustand 4: Oligomycin; entkoppelt: 2,4-dinitrophenol, carbonylcyanide p-trifluoromethoxyphenyl-hydrazine (FCCP)). Die Atmungsraten wurden auf den Gesamtproteingehalt normalisiert.

### Glukoseaufnahme-Test:

Die Glukoseaufnahme wurde mit murinen und humanen SKM-Zellen unter Verwendung des Glukoseaufnahmekits von abcam (ab136956) (abcam plc., Cambridge, UK) nach Herstellerangaben gemessen.

### Pharmakologische Aktivierung des Energieaufwands:

8-wöchige männliche C57BL/6 WT, A2B-/- und Wildtyp-Wurfgeschwister wurden fünf Minuten vor der Messung 2-({6-Amino-3,5-dicyano-4-[4-(cyclopropylmethoxy)phenyl]-pyridin-2-yl}sulfanyl)-acetamid (Bay 60-6583) subkutan injiziert. Der Sauerstoffverbrauch wurde mit PhenoMaster (TSE Systems GmbH, Bad Homburg, DE) alle 6 Minuten für 120 s gemessen. Zeitverlauf und relative Zunahme (bezogen auf das jeweilige t = 0) werden dargestellt.

### Isolierung von Satellitenzellen:

Satellitenzellen wurden aus Beinmuskeln von A2B+/+ und A2B-/- sowie aus WT-Mäusen isoliert, die wie beschrieben vier Wochen lang einmal täglich mit 2-({6-Amino-3,5-dicyano-4-[4-(cyclopropyl-methoxy)phenyl]-pyridin-2-yl}sulfanyl)-acetamid (Bay 60-6583) (1 mg/kg) behandelt wurden (N. Motohashi, Y. Asakura, A. Asakura, J. Vis. Exp. 2014, 86: e50846).

Nachfolgend werden die durch die Erfindung erzielten vorteilhaften Effekte beschrieben.

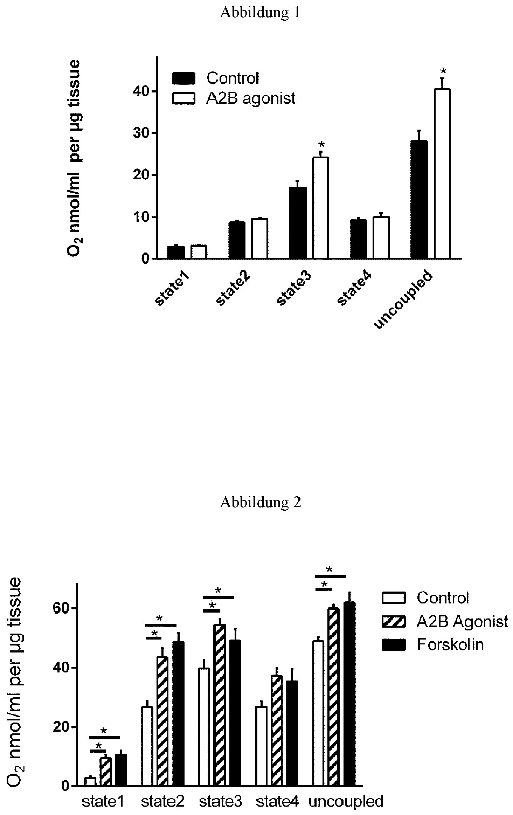

Abbildung 1 zeigt, dass der Sauerstoffverbrauch in Bezug auf die Gewebemasse (in humanen Skelettmuskel-Explantaten) durch die pharmakologische Stimulation des Adenosin-A_{2B}-Rezeptors mittels des Adenosin-A_{2B}-Rezeptor-Agonisten 2-({6-Amino-3,5-dicyano-4-[4-(cyclopropylmethoxy)-phenyl]pyridin-2-yl}sulfanyl)-acetamid (Bay 60-6583) signifikant gesteigert wird. (Zustand 1: endogen; Zustand 2: Adenosindiphosphat (ADP); Zustand 3: Succinat; Zustand 4: Oligomycin; entkoppelt: 2,4-Dinitrophenol, Carbonylcyanid p-trifluoromethoxyphenyl-hydrazin (FCCP))
Abbildung 2 zeigt, dass die pharmakologische Stimulation des Adenosin-A_{2B}-Rezeptors mit dem spezifischen Adenosin-A_{2B}-Agonisten 2-({6-Amino-3,5-dicyano-4-[4-(cyclopropylmethoxy)-phenyl]pyridin-2-yl}sulfanyl)-acetamid (Bay 60-6583) den Sauerstoffverbrauch von murinem Muskelgewebe *ex vivo* signifikant erhöht. (Zustand 1: endogen; Zustand 2: Adenosindiphosphat (ADP); Zustand 3: Succinat; Zustand 4: Oligomycin; entkoppelt: 2,4-Dinitrophenol, Carbonylcyanid p-trifluoromethoxyphenyl-hydrazin (FCCP))
Abbildung 3 zeigt, dass die Expression von Adenoisn-A_{2B}-Rezeptoren in humanem Skelettmuskel *ex vivo* mit dem basalen Sauerstoffverbrauch korreliert (mit und ohne Zusatz von Substrat (Succinat) zur Atmungskette).
Abbildung 4 zeigt, dass der Sauerstoffverbrauch in Bezug auf das veratmete Protein (in primären humanen Skelettmuskelzellen) durch die pharmakologische Stimulation des Adenosin-A_{2B}-Rezeptors mittels des Adenosin-A_{2B}-Rezeptor-Agonisten 2-({6-Amino-3,5-dicyano-4-[4-(cyclo-propylmethoxy)-phenyl]pyridin-2-yl}-sulfanyl)acetamid (Bay 60-6583) signifikant gesteigert wird. Forskolin ist eine positiv-Kontrolle und dient zum Vergleich. (Zustand 1: endogen; Zustand 2: Adenosindiphosphat (ADP); Zustand 3: Succinat; Zustand 4: Oligomycin; entkoppelt: 2,4-dinitrophenol, carbonylcyanide p-trifluoromethoxyphenyl-hydrazine (FCCP)).
Abbildung 5 zeigt, dass die Aufnahme von Glukose durch primäre humane Skelettmuskelzellen durch die pharmakologische Stimulation des Adenosin-A_{2B}-Rezeptors mittels des Adenosin-A_{2B}-Rezeptor-Agonisten 2-({6-Amino-3,5-dicyano-4-[4-(cyclopropylmethoxy)phenyl]-pyridin-2-yl}sulfanyl)-acetamid (Bay 60-6583) signifikant gesteigert werden kann.
Abbildung 6 zeigt, dass die Muskelmasse in Mäusen durch die pharmakologische Stimulation des Adenosin-A_{2B}-Rezeptors mittels des spezifischen Adenosin-A_{2B}-Rezeptor-Agonisten 2-({6-Amino-3,5-dicyano-4-[4-(cyclopropylmethoxy)-phenyl]pyridin-2-yl}sulfanyl)-acetamid (Bay 60-6583) signifikant gesteigert werden kann. Behandelte gealterte (20 Monate alte) Mäuse haben hierbei eine vergleichbare Muskelmasse wie junge (2 Monate alte) Kontrolltiere.
Abbildung 7 zeigt, dass die Muskelkraft von Mäusen mit einem Defizit an Adenosin-A_{2B}-Rezeptoren im Vergleich zu gleichaltrigen Wildtyp Mäusen signifikant verringert ist.
Abbildung 8 zeigt, dass die Muskelkraft von Mäusen durch die pharmakologische Stimulation des Adenosin-A_{2B}-Rezeptors mittels des spezifischen Adenosin-A_{2B}-Rezeptor-Agonisten 2-({6-Amino-3,5-dicyano-4-[4-(cyclopropylmethoxy)-phenyl]pyridin-2-yl}sulfanyl)-acetamid (Bay 60-6583) signifikant gesteigert wird. Obwohl die Muskelkraft im Alter nachlässt haben gealterte Mäuse haben hierbei so viel Kraft wie junge Kontrolltiere.
Abbildung 9 zeigt, dass die Menge an Satellitenzellen von alten Mäusen durch die pharmakologische Stimulation des Adenosin-A_{2B}-Rezeptors mittels des spezifischen Adenosin-A_{2B}-Rezeptor-Agonisten 2-({6-Amino-3,5-dicyano-4-[4-(cyclopropylmethoxy)-phenyl]pyridin-2-yl}-sulfanyl)acetamid (Bay 60-6583) signifikant erhöht werden kann.

## Patentansprüche

1. Ein Adenosin-A_{2B}-Rezeptor-Agonist zur Anwendung bei der Behandlung einer Krankheit ausgewählt aus Krankheiten des Muskel-Skelett-Systems und des Bindegewebes.

2. Der Adenosin-A_{2B}-Rezeptor-Agonist zur Anwendung nach Anspruch 1, wobei die Krankheit ausgewählt ist aus Krankheiten der Weichteilgewebe.

3. Der Adenosin-A_{2B}-Rezeptor-Agonist zur Anwendung nach Anspruch 2, wobei die Krankheit ausgewählt ist aus Krankheiten der Muskeln.

4. Der Adenosin-A_{2B}-Rezeptor-Agonist zur Anwendung nach Anspruch 3, wobei die Krankheit Sarkopenie ist.

5. Der Adenosin-A_{2B}-Rezeptor-Agonist zur Anwendung nach Anspruch 4, wobei die Sarkopenie ausgewählt ist aus der Gruppe bestehend aus altersabhängiger Sarkopenie, aktivitätsbedingter Sarkopenie, erkrankungsbedingter Sarkopenie, ernährungsbedingter Sarkopenie und Kombinationen davon.

6. Der Adenosin-A_{2B}-Rezeptor-Agonist zur Anwendung nach einem der vorstehenden Ansprüche, wobei der Adenosin-A_{2B}-Rezeptor-Agonist ausgewählt ist aus der Gruppe bestehend aus 2-({6-Amino-3,5-dicyano-4-[4-(cyclopropylmethoxy)phenyl]pyridin-2-yl}sulfanyl)-acetamid (BAY 60-6583), 1-(6-Amino-9H-purin-9-yl)-1-deoxy-N-ethyl-β-D-ribo-furanuronamid (NECA), 9-β-D-Ribofuranosyladenin (Adenosin), 2-Amino-4-(4-hydroxyphenyl)-6-(1H-imidazol-2-ylmethyl-sulfanyl)pyridin-3,5-dicarbonitril (LUF-5834), 6-Amino-2-chloropurin ribosid (CADO) und deren physiologisch verträglichen Salzen und/oder Solvaten.

7. Der Adenosin-A_{2B}-Rezeptor-Agonist zur Anwendung nach einem der vorstehenden Ansprüche, wobei die Krankheit Sarkopenie ist und wobei der Adenosin-A_{2B}-Rezeptor-Agonist 2-({6-Amino-3,5-dicyano-4-[4-(cyclopropylmethoxy)phenyl]pyridin-2-yl}sulfanyl)-acetamid oder ein physiologisch verträgliches Salz und/oder Solvat davon ist.

8. Der Adenosin-A_{2B}-Rezeptor-Agonist zur Anwendung nach einem der vorstehenden Ansprüche, wobei der Adenosin-A_{2B}-Rezeptor-Agonist systemisch oder lokal verabreicht wird.

9. Der Adenosin-A_{2B}-Rezeptor-Agonist zur Anwendung nach einem der vorstehenden Ansprüche, wobei der Adenosin-A_{2B}-Rezeptor-Agonist oral, transdermal, intravenös, subkutan, intraperitoneal oder rektal verabreicht wird.

10. Der Adenosin-A_{2B}-Rezeptor-Agonist zur Anwendung nach einem der vorstehenden Ansprüche, wobei der Adenosin-A_{2B}-Rezeptor-Agonist einmal täglich, zweimal täglich oder dreimal täglich verabreicht wird.

11. Der Adenosin-A_{2B}-Rezeptor-Agonist zur Anwendung nach Anspruch 3 oder 4, wobei die Sarkopenie ausgewählt ist aus der Gruppe bestehend aus Sarkopenie
- der Schulterregion, bevorzugt Klavikula, Skapula, Akromioklavikular-, Schulter- und/oder Sternoklavikulargelenk,
- des Oberarms, bevorzugt Humerus und/oder Ellenbogengelenk,
- des Unterarms, bevorzugt Radius, Ulna und/oder Handgelenk,
- der Hand, bevorzugt Finger, Handwurzel, Mittelhand und/oder Gelenke zwischen diesen Knochen,
- der Beckenregion oder Oberschenkel, bevorzugt Becken, Femur, Gesäß, Hüfte, Hüftgelenk und/oder Iliosakralgelenk,
- der Unterschenkel, bevorzugt Fibula, Tibia und/oder Kniegelenk,
- der Knöchel und des Fußes, bevorzugt Fußwurzel, Mittelfuß, Zehen, Sprunggelenk und/oder sonstige Gelenke des Fußes, und
- sonstiger Lokalisationen, bevorzugt Hals, Kopf, Rippen, Rumpf, Schädel und/oder Wirbelsäule.

12. Eine Substanz, welche den Adenosin-A_{2B}-Rezeptor agonisiert, zur Anwendung
- bei der Erhöhung des Sauerstoffverbrauchs von primären humanen Skelettmuskelzellen, und/oder
- bei der Erhöhung der Glukoseaufnahme von primären humanen Skelettmuskeln,
im menschlichen Körper,
bevorzugt bei der Behandlung und/oder Linderung einer Krankheit ausgewählt aus Krankheiten des Muskel-Skelett-Systems und des Bindegewebes.

13. Die Substanz zur Anwendung nach Anspruch 12, wobei die Krankheit Sarkopenie ist.

14. Die Substanz zur Anwendung nach Anspruch 12 oder 13, wobei die Substanz einen IC50 Wert von höchstens 100 µM, bevorzugter höchstens 10 µM, noch bevorzugter höchstens 1000 nM, am bevorzugtesten höchstens 500 nM, insbesondere höchstens 250 mM aufweist.

15. Die Substanz zur Anwendung nach Anspruch 12 oder 13, wobei die Substanz 2-({6-Amino-3,5-dicyano-4-[4-(cyclopropylmethoxy)phenyl]pyridin-2-yl}sulfanyl)-acetamid oder ein physiologisch verträgliches Salz und/oder Solvat davon ist.
